# EUROPEAN PATENT APPLICATION

(11) **EP 4 289 962 A2**
(43) Date of publication of application: **13.12.2023**
(21) Application number: 22750032.9
(22) Date of filing: 04.02.2022
(51) Int. Cl.: C12P 19/04, C12N 1/20, A23L 33/125, A61K 35/747, A61K 31/715, A61P 29/00, A61P 3/00, A61P 1/16, A61K 31/7004, C12R 1/25

(54) **COMPOSITION COMPRISING LACTOBACILLUS PLANTARUM-DERIVED POLYSACCHARIDE OR EXTRACT**

(30) Priority: 04.02.2021 KR 20210016012
(71) Applicant: Neoregen Biotech, Suwon-si, Gyeonggi-do 16614 (KR)
(72) Inventor: SEO, Jeong Min, Seoul 06587 (KR); LEE, Jae Hoon, Seoul 07774 (KR)
(74) Representative: dompatent von Kreisler Selting Werner - Partnerschaft von Patent- und Rechtsanwälten mbB
(86) International application number: PCT/KR2022/001731
(87) International publication number: WO 2022/169283

(57) **Abstract**

The present invention relates to a composition including *Lactobacillus plantarum*-derived polysaccharide or extract, and more specifically, by including the *Lactobacillus plantarum*-derived polysaccharide or the *Lactobacillus plantarum* extract, may exhibit inflammatory inhibition, adipogenesis inhibition, intracellular glucose absorption inhibition, insulin resistance reduction and hepatic steatosis reduction effects, and may exhibit effects of treating or preventing a metabolic disease.

## Description

### [Technical Field]

The present invention relates to a composition including a polysaccharide derived from *Lactobacillus plantarum* or an extract of *Lactobacillus plantarum.*

### [Background Art]

According to the World Health Organization (WHO), probiotics are defined as "living microorganisms that confer a health benefit on the host when administered in appropriate doses." The probiotics market is expected to grow by 37% globally from 2016 to 2020 due to effects of improving gut health and preventing insulin resistance. However, recent studies have shown that regular consumption of probiotics may lead to unexpected side effects. For example, administration of probiotics may result in infection, unwanted inflammatory responses, and gene transfer from the probiotics to the natural host microorganism.

In recent years, interest in postbiotics is increasing in order to minimize the side effects of probiotics as described above. Postbiotics, also known as "simple metabolites" or "cell-free supernatants," are identified as bioactive compounds secreted by living lactic acid bacteria (LABs). Postbiotics may include functional bioactive compounds such as metabolites, functional proteins, cell lysates and short chain fatty acids. Postbiotics may be used as a substitute for probiotics because they can exhibit probiotic effects without the risk of transferring the antibiotic resistance gene to the host. Postbiotics may be recommended for children under 5 years of age because the risk of LAB-associated infections in infants and young children, such as pneumonia and meningitis, is rare. Further, postbiotics are stable over a wide range of pH and temperature and can be separated into individual components, thereby making the same suitable for *in vitro* and *in vivo* studies and easy for commercialization. In a recent study, exopolysaccharide (EPS), one of the postbiotics secreted by lactic acid bacteria, showed antiviral efficacy in intestinal epithelial cells.

### [Summary of Invention]

### [Problems to be Solved by Invention]

An object of the present invention to provide a *Lactobacillus plantarum*-derived polysaccharide or a *Lactobacillus plantarum* extract.

In addition, another object of the present invention is to provide a pharmaceutical composition for preventing or treating metabolic diseases, which includes a *Lactobacillus plantarum*-derived polysaccharide or a *Lactobacillus plantarum* extract.

Further, another object of the present invention is to provide a food composition for preventing or improving metabolic diseases, which includes a *Lactobacillus plantarum*-derived polysaccharide or a *Lactobacillus plantarum* extract.

### [Means for Solving Problems]

1. A pharmaceutical composition for preventing or treating a metabolic disease, including a *Lactobacillus plantarum*-derived polysaccharide or a *Lactobacillus plantarum* extract.
2. The pharmaceutical composition according to the above 1, wherein the *Lactobacillus plantarum* is at least one selected from the group consisting of *Lactobacillus plantarum* L-14 (accession number KCTC13497BP), *Lactobacillus plantarum* ATCC 10241, *Lactobacillus plantarum* NCDO704, and *Lactobacillus plantarum* NCDO1193.
3. The pharmaceutical composition according to the above 1, wherein the polysaccharide is at least one of an intracellular polysaccharide and an extracellular polysaccharide.
4. The pharmaceutical composition according to the above 1, wherein the polysaccharide is glucose.
5. The pharmaceutical composition according to the above 1, wherein the Lactobacillus plantarum extract is an ultrasonic crushed product of Lactobacillus plantarum.
6. The pharmaceutical composition according to the above 1, wherein the metabolic disease is at least one selected from the group consisting of insulin resistance, type 2 diabetes, hyperlipidemia, fatty liver, obesity and inflammation.
7. A food composition for preventing or improving a metabolic disease, including a *Lactobacillus plantarum-*derived polysaccharide or a *Lactobacillus plantarum* extract.

### [Advantageous effects]

The *Lactobacillus plantarum*-derived polysaccharide or the *Lactobacillus plantarum* extract of the present invention may suppress the inflammatory response. The *Lactobacillus plantarum*-derived polysaccharide or the *Lactobacillus plantarum* extract of the present invention may exhibit anti-inflammatory efficacy by inhibiting an interaction between LPS and TLR4.

Further, the *Lactobacillus plantarum*-derived polysaccharide or the *Lactobacillus plantarum* extract of the present invention may exhibit effects of inhibiting adipogenesis, inhibiting intracellular glucose uptake, reducing insulin resistance and reducing hepatic steatosis, and further exhibit effects of improving the metabolic disease. The *Lactobacillus plantarum*-derived polysaccharide or the *Lactobacillus plantarum* extract of the present invention may interact with TLR2 to activate AMPK pathway, thereby preventing adipogenesis.

### [Brief Description of Drawings]

FIG. 1 shows results of analyzing characteristics of *Lactobacillus plantarum*-derived extracellular polysaccharides according to an embodiment.
FIG. 2 shows results of confirming the inflammatory response inhibitory efficacy of metabolites secreted from *Lactobacillus plantarum* L-14 strain in mouse macrophages.
FIG. 3 shows results of confirming the efficacy of *Lactobacillus plantarum*-derived EPS according to an embodiment for inhibiting the morphological change of LPS-induced cells.
FIG. 4 shows results of confirming the efficacy of *Lactobacillus plantarum*-derived EPS according to an embodiment for inhibiting LPS-induced inflammatory response.
FIG. 5 shows results of confirming the efficacy of *Lactobacillus plantarum*-derived EPS according to an embodiment for inhibiting nuclear translocation of LPS-induced NF-κB.
FIG. 6 shows results of confirming that the *Lactobacillus plantarum*-derived EPS according to an embodiment regulates MAPK and NRF2/HO-1 pathways, which are major inflammatory response control pathways.
FIG. 7 shows results of confirming that the *Lactobacillus plantarum*-derived EPS according to an embodiment inhibits the inflammatory response through a TLR4 pathway.
FIG. 8 shows results of confirming the efficacy of the *Lactobacillus plantarum* extract according to an embodiment for inhibiting differentiation from preadipocytes to mature adipocytes.
FIG. 9 shows results of confirming the efficacy of the *Lactobacillus plantarum* extract according to an embodiment for inhibiting weight gain and the expression of inflammatory markers in a high-fat diet mouse model.
FIG. 10 shows results of confirming the efficacy of the *Lactobacillus plantarum* extract according to an embodiment for inhibiting insulin resistance marker and liver fat.
FIG. 11 shows results of confirming an AMPK signal transduction pathway up-regulation effect and an adipogenesis inhibitory effect of the *Lactobacillus plantarum* extract according to an embodiment.
FIG. 12 shows an inhibitory effect of the *Lactobacillus plantarum* extract according to an embodiment on the differentiation of human bone marrow mesenchymal stem cells (BM-MSC) into adipocytes.
FIG. 13 shows results of confirming an adipogenesis inhibitory effect of the *Lactobacillus plantarum* extract according to an embodiment under severe temperature conditions or pH change conditions.
FIG. 14 shows results of confirming an adipogenesis inhibitory efficacy of the *Lactobacillus plantarum*-derived polysaccharide according to an embodiment.
FIG. 15 shows results of confirming the adipogenesis inhibitory efficacy of the *Lactobacillus plantarum*-derived polysaccharide according to an embodiment.
FIG. 16 shows results of confirming the adipogenesis inhibitory efficacy of the *Lactobacillus plantarum* extract according to an embodiment.

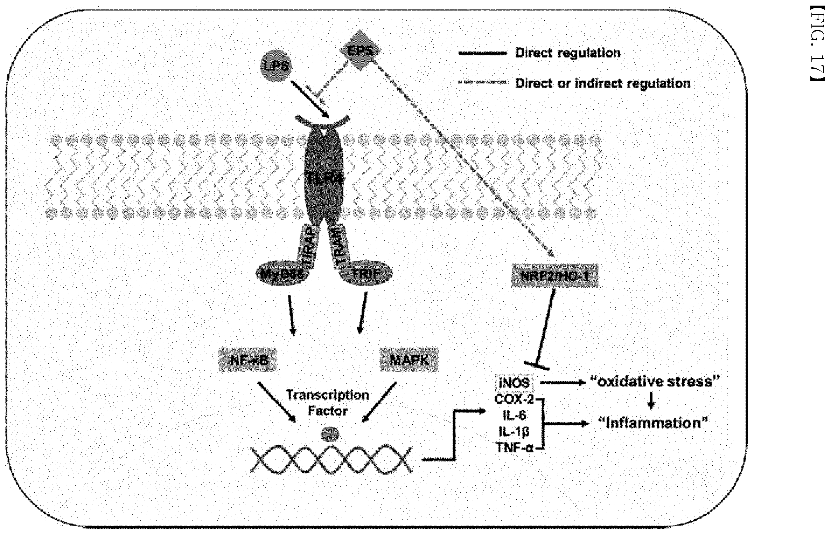

FIG. 17 is a diagram schematically illustrating that the *Lactobacillus plantarum*-derived polysaccharide inhibits TLR4 and MyD88 signaling to suppress pro-inflammatory mediators such as NF-B and MAPK pathways.

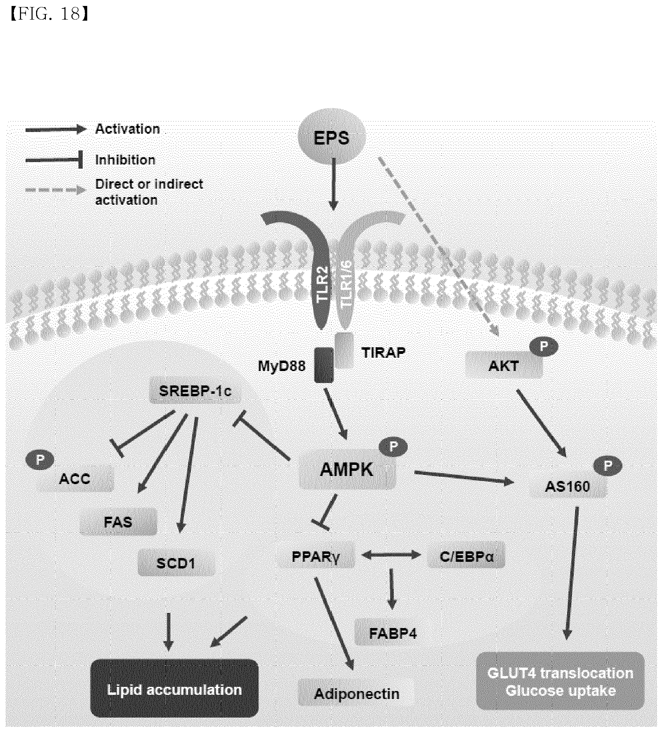

FIG. 18 is a diagram schematically illustrating that *Lactobacillus plantarum*-derived polysaccharide regulates lipid accumulation and glucose uptake through TLR2 and AMPK signaling pathways.
FIG. 19 shows FPLC results of analyzing the characteristics of the *Lactobacillus plantarum*-derived polysaccharide according to an embodiment.

### [Mode for Carrying out Invention]

The present invention provides a pharmaceutical composition for preventing or treating inflammatory or metabolic diseases, which includes a *Lactobacillus plantarum*-derived polysaccharide or a *Lactobacillus plantarum* extract.

The *Lactobacillus plantarum*-derived polysaccharide may be one of postbiotics. The term "postbiotics" refer to substances produced by the metabolism, fermentation, etc. of probiotics, and may include short-chain fatty acids, antibacterial peptides, vitamin B, vitamin K, complex amino acids, peptides, neurotransmitters, enzymes, minerals, teichoic acid, polysaccharides, cell surface proteins, etc., which may each exhibit different functions.

Since probiotics such as *Lactobacillus plantarum* bacteria and a culture solution containing the bacteria include living bacteria, there is a possibility that safety problems such as bacteremia may appear in an individual with weakened immunity, whereas *Lactobacillus plantarum-*derived polysaccharides may avoid side effects possibly appearing due to inclusion of bacteria, and may exhibit excellent safety. Further, in the case of probiotics, the number of living bacteria (CFU) is not maintained constant from the time of production to the expiration date but is continuously reduced such that it may be difficult to maintain their functionality, whereas, for *Lactobacillus plantarum*-derived polysaccharides, a dose of the ingredient expressing the functionality at the time of production is maintained constant such that the functionality can be maintained consistently. Specifically, *Lactobacillus plantarum*-derived polysaccharides are not affected by a change in the external environment such as heat, humidity, and pH, etc. compared to *Lactobacillus plantarum* bacteria or a culture solution containing the same such that the functionality can be maintained under various conditions. Therefore, the polysaccharide isolated from *Lactobacillus plantarum* is suitable for commercialization as a product. According to one embodiment, it was confirmed that the *Lactobacillus plantarum*-derived polysaccharide showed stability without being affected by external environmental changes such as heat, humidity, and pH, etc.

*Lactobacillus plantarum* may be a known strain, and may be, for example, at least one selected from the group consisting of *Lactobacillus plantarum* L-14 (accession number KCTC13497BP), *Lactobacillus plantarum* ATCC 10241, *Lactobacillus plantarum* NCDO704, and *Lactobacillus plantarum* NCDO1193, but it is not limited thereto.

*Lactobacillus plantarum* L-14 (accession number KCTC13497BP) was deposited with the Biological Resource Center of the Korea Research Institute of Bioscience and Biotechnology on March 15, 2018, of which the accession number is KCTC13497BP. Name of deposit institution: Korea Research Institute of Bioscience and Biotechnology, accession number: KCTC13497BP, deposit date: 20180315.

The term "polysaccharide" refers collectively to saccharides in which three or more monosaccharides form large molecules through glycosidic bonds, which become monosaccharides when hydrolyzed.

The type of polysaccharide is not limited as long as it is a high-molecular polysaccharide produced and discharged by microorganisms including lactic acid bacteria during the growth process. For example, the polysaccharide may be an extracellular polysaccharide (exopolysaccharides, EPS), an intracellular polysaccharide, or a structural polysaccharide. Specifically, the polysaccharide is an extracellular polysaccharide or an intracellular polysaccharide.

For example, the polysaccharide may be recovered from a culture solution of *Lactobacillus plantarum.* The polysaccharide is a metabolite discharged from *Lactobacillus plantarum* into the culture solution during fermentation, and may be recovered from the culture solution. According to one embodiment, the polysaccharide may be separated from a culture solution obtained by culturing the *Lactobacillus plantarum* strain in a medium. Specifically, the polysaccharide may be separated by denaturing a protein in the culture solution from which the strain is removed then removing the same, and adding ethanol thereto.

The *Lactobacillus plantarum*-derived polysaccharide may be isolated from the *Lactobacillus plantarum* strain extract. For example, the polysaccharide may be isolated from the lysate of the *Lactobacillus plantarum* strain. According to one embodiment, the polysaccharide may be isolated by denaturing the protein in the extract (also called as a lysate) obtained by ultrasonically crushing the *Lactobacillus plantarum* strain, then collecting the supernatant, followed by adding ethanol thereto.

The production of polysaccharides using microorganisms may be influenced by environmental factors such as culture time, culture pH, culture temperature, O₂ concentration, and agitation, etc., as well as the type and concentration of carbon source, type and concentration of nitrogen source, the content of nutrients such as phosphoric acid, sulfur, potassium, magnesium, iron, calcium, etc., and the culture method.

The *Lactobacillus plantarum*-derived polysaccharide is sufficient as long as it is derived from *Lactobacillus plantarum,* and the type of strain is not limited.

The *Lactobacillus plantarum*-derived polysaccharide may be a homogenous polysaccharide.

The *Lactobacillus plantarum*-derived polysaccharide may be glucose.

The *Lactobacillus plantarum*-derived polysaccharide may be a glucose homogeneous-polysaccharide.

The *Lactobacillus plantarum*-derived polysaccharide may have a weight average molecular weight (Mw) of 3 × 10⁴ to 12 × 10⁴ Da, 4 × 10⁴ to 11 × 10⁴ Da, 5 × 10⁴ to 10 × 10⁴ Da, 6 × 10⁴ to 9 × 10⁴ Da, or 7 × 10⁴ to 8 × 10⁴ Da. According to one embodiment, the *Lactobacillus plantarum-*derived polysaccharide may have a weight average molecular weight of 7.57 × 10⁴ Da.

The *Lactobacillus plantarum*-derived polysaccharide may have a number average molecular weight (Mn) of 0.01 × 10⁴ to 6 × 10⁴ Da, 0.05 × 10⁴ to 5 × 10⁴ Da, 0.1 × 10⁴ to 4 × 10⁴ Da, 0.5 × 10⁴ to 3 × 10⁴ Da, or 1 × 10⁴ to 2 × 10⁴ Da. According to one embodiment, the *Lactobacillus plantarum-*derived polysaccharide may have a number average molecular weight of 1.84 × 10⁴ Da.

The *Lactobacillus plantarum*-derived polysaccharide may have a polydispersity index (PDI) of 2.5 to 6, 3 to 5.5, 3.5 to 5, or 4 to 4.5. According to one embodiment, the *Lactobacillus plantarum*-derived polysaccharide may have a polydispersity index of 4.12.

In one embodiment, the intrinsic properties of polysaccharides isolated from *Lactobacillus plantarum* are analyzed by fast protein liquid chromatography (FPLC), thin layer chromatography (TLC), Fourier-transform infrared spectroscopy (FTIR), and gel permeation chromatography (GPC), etc.

The *Lactobacillus plantarum*-derived polysaccharide of the present invention exhibits excellent anti-inflammatory effect. According to one embodiment, the *Lactobacillus plantarum*-derived polysaccharide may suppress inflammatory cytokines. For example, the *Lactobacillus plantarum-*derived extracellular polysaccharide may reduce expression levels of IL-6, TNF-α and IL-1α, etc. According to one embodiment, the *Lactobacillus plantarum*-derived polysaccharide may reduce the expression level of COX-2 and inducible nitric oxide synthase (iNOS), which are known as major mediators of inflammation. According to one embodiment, the *Lactobacillus plantarum*-derived polysaccharide may inhibit phosphorylation of NF-κB and translocation to the nucleus. According to one embodiment, the *Lactobacillus plantarum*-derived polysaccharide may inhibit phosphorylation of MAPK family proteins, such as JNK, ERK or p38, etc. According to one embodiment, the *Lactobacillus plantarum*-derived polysaccharide may inhibit the expression of nuclear factor E2-related factor 2 (NRF2) and heme oxygenase-1 (HO-1). The *Lactobacillus plantarum-*derived polysaccharide of the present invention may exhibit anti-inflammatory efficacy by inhibiting an interaction between LPS and TLR4.

The *Lactobacillus plantarum*-derived polysaccharide of the present invention may exhibit excellent anti-obesity effect. According to one embodiment, the *Lactobacillus plantarum*-derived polysaccharide may inhibit the differentiation of preadipocytes into adipocytes. According to one embodiment, the *Lactobacillus plantarum-*derived polysaccharide may exhibit an effect of inhibiting adipogenesis and intracellular glucose uptake. The *Lactobacillus plantarum*-derived polysaccharide of the present invention may inhibit adipogenesis by activating the AMPK pathway through interaction with TLR2.

The *Lactobacillus plantarum*-derived polysaccharide of the present invention may exhibit excellent insulin resistance or liver fat inhibitory effect. According to one embodiment, the *Lactobacillus plantarum*-derived polysaccharide may reduce fasting blood sugar, fasting insulin, leptin, and resistin, while inhibiting fat accumulation in the liver.

The *Lactobacillus plantarum*-derived polysaccharide of the present invention may be purified using an alcohol precipitation method.

For example, the *Lactobacillus plantarum*-derived polysaccharide may be obtained by a method including adding acetic acid to the *Lactobacillus plantarum* culture solution, and then adding alcohol thereto to separate the precipitate.

For another example, the *Lactobacillus plantarum-*derived polysaccharide may be obtained by a method including adding acetic acid to the *Lactobacillus plantarum* extract and then adding alcohol thereto to separate the precipitate.

The method may further include dialysis performed by inputting purified water into the precipitate after the separation of the precipitate by adding alcohol.

The acetic acid may be trichloroacetic acid. A content of protein in the culture solution may be denatured by treatment with acetic acid.

The alcohol may be C1 to C4 alcohol, and according to an embodiment, may be ethanol. Nucleic acid may be degraded by addition of alcohol. The alcohol may be absolute ethanol.

The *Lactobacillus plantarum* extract may be a lysate of *Lactobacillus plantarum,* and may be, for example, powder, solution or dispersion thereof, which is obtained by freeze-drying the lysate of *Lactobacillus plantarum.*

The *Lactobacillus plantarum* extract may be an ultrasonic crushed product of *Lactobacillus plantarum.*

If the *Lactobacillus plantarum* extract is an extract obtained by sonicating a *Lactobacillus plantarum* strain with an ultrasonicator, the type of the strain is not limited. According to one embodiment, the *Lactobacillus plantarum* extract may be a *Lactobacillus plantarum* L-14 extract, a *Lactobacillus plantarum* ATCC10241 extract, a *Lactobacillus plantarum* NCDO704 extract, or a *Lactobacillus plantarum* NCDO1193 extract.

The *Lactobacillus plantarum* extract may be obtained by sonicating *Lactobacillus plantarum* cultured in a culture medium. The *Lactobacillus plantarum* extract may be obtained by removing cell wall components and other residues from the cultured and sonicated *Lactobacillus plantarum.* Sonication may be performed at 0 °C or lower.

The *Lactobacillus plantarum* extract of the present invention may exhibit an excellent anti-inflammatory effect. According to one embodiment, the *Lactobacillus plantarum* extract may reduce the expression of inflammatory markers such as leptin, interleukin-6 (IL-6), tumor necrosis factor-α (TNF-α), and resistin in the liver or adipose tissue, and may increase the expression of anti-inflammatory markers such as adiponectin and arginase 1 (Arg1).

The *Lactobacillus plantarum* extract of the present invention may exhibit excellent anti-obesity effect. According to one embodiment, the *Lactobacillus plantarum* extract may inhibit the differentiation of preadipocytes into adipocytes. According to one embodiment, the *Lactobacillus plantarum* extract may exhibit effects of inhibiting adipogenesis and inhibiting intracellular glucose uptake.

The *Lactobacillus plantarum* extract of the present invention may exhibit excellent insulin resistance or liver fat inhibitory effect. According to one embodiment, the *Lactobacillus plantarum* extract may reduce fasting blood sugar, fasting insulin, leptin and resistin, and may inhibit fat accumulation in the liver.

The metabolic disease may be at least one selected from the group consisting of insulin resistance, type 2 diabetes, hyperlipidemia, fatty liver, obesity and inflammation, but it is not limited thereto.

The pharmaceutical composition may be administered in the form of various oral and parenteral formulations, and in the case of formulation, diluents or excipients such as fillers, extenders, binders, humectants, disintegrants, surfactants, and the like may be usually used to prepare the formulation.

The term "treatment" refers to a procedure that includes some extent of alleviation including slight alleviation, substantial alleviation or major relaxation as well as healing, and leads to beneficial effects on a subject or patient suffering from a disease condition to be treated, wherein the extent of relaxation means at least slight relaxation.

The term "prevention" refers to a precautionary procedure that includes a slight, substantial or significant reduction in possibility of occurrence or recurrence of disease condition as well as overall prevention, and leads to a reduction in some extent of onset possibility of the disease condition to be prevented or the disease condition recurred or being recurred, wherein the extent of reduction in possibility means at least slight reduction.

Further, the present invention provides a food composition for preventing or improving inflammatory or metabolic diseases, which includes a *Lactobacillus plantarum*-derived polysaccharide or a *Lactobacillus plantarum* extract.

The *Lactobacillus plantarum*-derived polysaccharide, the *Lactobacillus plantarum* extract, and the inflammatory or metabolic disease have been described above, and therefore will not be described in detail.

The form of the food composition is not particularly limited, and may be, for example, drinks, meat, sausage, bread, biscuits, rice cakes, chocolate, candy, snacks, confectionery, pizza, ramen, other noodles, gums, dairy products including ice cream, various soups, beverages, alcoholic beverages and vitamin complexes, milk products, processed milk products and the like.

Further, the present invention provides a method for preventing or treating inflammatory or metabolic diseases, which includes administering to an individual a *Lactobacillus plantarum*-derived polysaccharide or a *Lactobacillus plantarum* extract.

The *Lactobacillus plantarum*-derived polysaccharide, the *Lactobacillus plantarum* extract, and the inflammatory or metabolic disease have been described above, and therefore will not be described in detail.

The subject may be a mammal such as a human, cow, horse, pig, dog, sheep, goat, or cat.

Administration may be conducted by any method known in the art. Further, administration may be conducted directly to a subject by any means, for example, through different routes such as oral, intravenous, intramuscular, transdermal, mucosal, intranasal, intratracheal or subcutaneous administration. Further, administration may be conducted systemically or locally. Moreover, administration may be topical administration.

Hereinafter, the configuration and effects of the present invention will be described in more detail by way of examples. However, the following examples are provided for illustrative purposes only to help understanding of the present invention, and the scope and range of the present invention are not limited thereto.

### I. Preparation of Lactobacillus plantarum extracts

### 1. Example 1: Lactobacillus plantarum L-14 extract

*Lactobacillus plantarum* L-14 strain (also referred to as L-14 in the description of the present invention) obtained from Neorigen Biotech (Gyeonggi-do, Korea) (KTCT13497BP) was pre-cultured in MRS medium at 37 °C for 18 hours. Then, these were inoculated 1% in 500 mL MRS broth and incubated at 37 °C for 18 hours. The cultured L-14 was harvested by a centrifuge (10,000 g at 4 °C for 10 minutes) and washed twice with PBS. Thereafter, MRS broth and PBS were completely removed by washing the harvest with distilled water. L-14 resuspended in 20 mL distilled water was sonicated for 30 minutes on ice using a sonicator. Pellets were discarded after centrifugation at 10,000 g at 4 °C for 20 minutes to remove cell wall components and other residues. The supernatant was filtered (0.2 um) and frozen overnight at -80 °C. Then, it was freeze-dried to obtain an L-14 extract (Example 1). The obtained L-14 extract was reconstituted with PBS before use. Further, the L-14 extract (N60, Example 2-1) was adjusted to pH 7.0 (hereinafter P60) or incubated at 90 °C for 30 minutes (hereinafter H60), thereby confirming the properties of effective molecules in the L-14 extract.

### 2. Example 2: Lactobacillus plantarum ATCC10241 extract

A *Lactobacillus plantarum* ATCC10241 extract (Example 2) was prepared by the same preparation method as described in Example 1, except that *Lactobacillus plantarum* subsp. plantarum (strain number: ATCC10241) was used instead of *Lactobacillus plantarum* L-14 strain (KTCT13497BP).

### 3. Example 3: Lactobacillus plantarum NCDO704 extract

A *Lactobacillus plantarum* NCDO704 extract (Example 3) was prepared by the same preparation method as described in Example 1, except that *Lactobacillus plantarum* (strain number: NCDO704) was used instead of *Lactobacillus plantarum* L-14 strain (KTCT13497BP).

### 4. Example 4: Lactobacillus plantarum NCDO1193 extract

A *Lactobacillus plantarum* NCDO1193 extract (Example 4) was prepared by the same preparation method as described in Example 1, except that *Lactobacillus plantarum* (strain number: NCDO1193) was used instead of *Lactobacillus plantarum* L-14 strain (KTCT13497BP).

### II. Preparation of Lactobacillus plantarum-derived polysaccharides

### 1. Example 5: Lactobacillus plantarum L-14 (KTCT13497BP)-derived extracellular polysaccharide

*Lactobacillus plantarum* L-14 strain (KTCT13497BP, hereinafter also referred to as L-14) obtained from Neorigen Biotech (Suwon, Gyeonggi-do) was incubated with MRS medium (Hardy Diagnostics, Santa Maria, CA, USA) containing 2% dextrose, 1% animal tissue peptic digest, 1% beef extract, 0.5% yeast extract, 0.5% sodium acetate, 0.2% disodium phosphate, 0.2% ammonium citrate, 0.1% polysorbate 80, 0.01% magnesium sulfate, 0.005% manganese, and 0.005% sulphate at 30 °C for 18 hours. The L-14 culture medium was separated via centrifugation at 10,000 g for 20 minutes. Then, the medium supernatant was separated, and trichloroacetic acid was added to denaturate the protein of the L-14 culture medium at 37 °C for 1 hour. Then, in order to remove the denatured protein, centrifugation was performed at 10,000 g for 20 minutes and only the supernatant was mixed with absolute ethanol. The resulting precipitate after mixing with absolute ethanol was recovered, followed by dialysis using distilled water (D.W.) at 4 °C for 24 to 48 hours in order to completely remove the medium components and other materials. Then, the dialyzed solution was lyophilized under reduced pressure to obtain L-14-derived EPS (Example 5), and for subsequent experiments, the EPS was resuspended in distilled water and stored at -80 °C.

### 2. Example 6: Lactobacillus plantarum L-14 (KTCT13497BP)-derived polysaccharide

The protein content was denatured by adding trichloroacetic acid to the L-14 extract (Example 1) prepared according to the method of the above I.1. to reach a final concentration of 14% (v/v). The L-14 extract was incubated at 37 °C for 30 minutes in a shaking incubator at 90 rpm and centrifuged at 8,000 g for 20 minutes. The supernatant was collected followed by adding cold absolute ethanol thereto to reach a final concentration of 67% (v/v). The mixture was incubated at 4 °C for 24 hours and the precipitate was collected. The same volume of D.W. as the precipitate was added. The solution was dialyzed using standard RC tubing (molecular weight cut-off: 3.5 kDa; Spectrum Chemical, New Brunswick, NJ, USA) while changing water twice a day for 2 days, and the dialysate was filtered (0.2 um). Then, it was freeze-dried to obtain an L-14-derived polysaccharide (Example 6). The obtained product in Example 6 was reconstituted with PBS before use.

### 3. Example 7: Lactobacillus plantarum ATCC10241-derived polysaccharide

A *Lactobacillus plantarum* ATCC10241-derived polysaccharide (Example 7) was prepared by the same method as described in Example 6 above, except that the extract (Example 2) prepared by the method of the above I.2. was used instead of the L-14 extract (Example 1) prepared by the method of the above I.1.

### 4. Example 8: Lactobacillus plantarum NCDO704-derived polysaccharide

A *Lactobacillus plantarum* NCDO704-derived polysaccharide (Example 8) was prepared by the same method as described in Example 6 above, except that the extract (Example 3) prepared by the method of the above I.3. was used instead of the L-14 extract (Example 1) prepared by the method of the above I.1.

### 5. Example 9: Lactobacillus plantarum NCDO1193 derived polysaccharide

A *Lactobacillus plantarum* NCDO1193-derived polysaccharide (Example 9) was prepared by the same method as described in Example 6 above, except that the extract (Example 4) prepared by the method of the above I.4. was used instead of the L-14 extract (Example 1) prepared by the method of the above I.1.

### III. Analysis of Lactobacillus plantarum-derived polysaccharides

### 1. Fast protein liquid chromatography (FPLC)

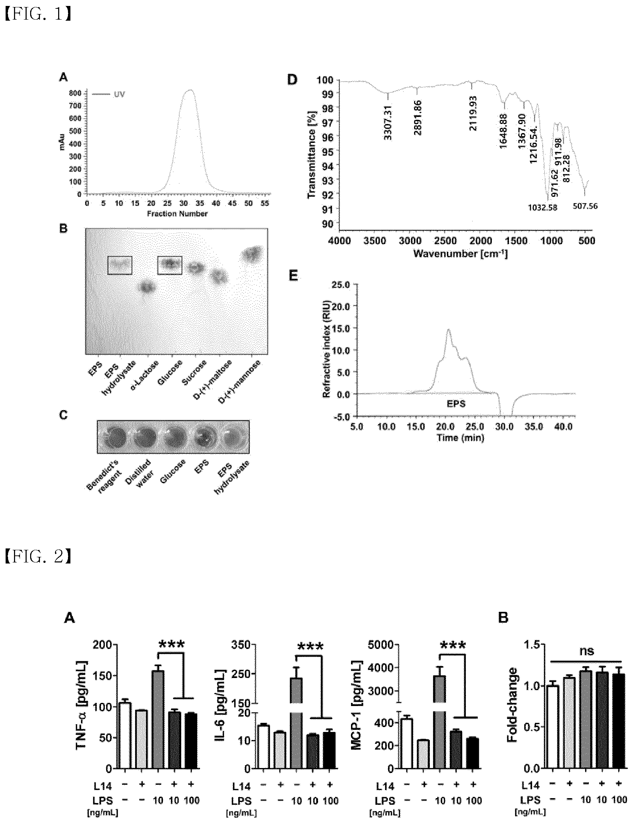

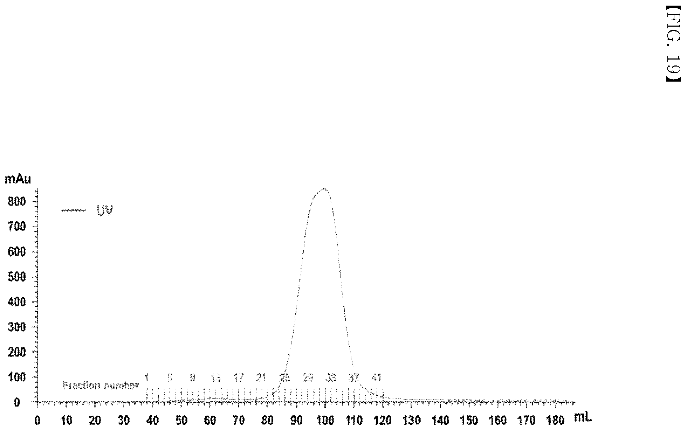

In order to identify whether the *Lactobacillus plantarum*-derived polysaccharides separated by the method of the above II are homogeneous polysaccharides, the polysaccharides were analyzed by fast protein liquid chromatography (FPLC) size exclusion chromatography. Specifically, EPS (30 mg/mL) was separated by size exclusion chromatography using PBS on a HiLoad^{®} 16/600 Superdex 200 pg column (GE Healthcare), and analyzed by AKTA fast protein liquid chromatography (GE Healthcare). As a result, the separated *Lactobacillus plantarum*-derived polysaccharide has generated a single symmetrical peak. This indicates that the polysaccharide is a homogeneous polysaccharide (FIG. 1A (Example 5) and FIG. 19 (Example 6)).

### 2. Thin layer chromatography (TLC) and Benedict's test

In order to determine the monosaccharide composition of the polysaccharides separated by the method of the above II, 10 mg of polysaccharide was hydrolyzed with 1 mL sulfuric acid (2N) at 100 °C for 4 hours. Residual sulfuric acid was neutralized with sufficient BaCO₃ for 12 hours. The EPS hydrolyzate was adjusted to pH 7 and then lyophilized for analysis. The EPS hydrolyzate was treated on TLC silica gel (Merck, Darmstadt, Germany) and transferred to a buffer consisting of n-butanol : methanol : 25% ammonia solution : D.W. (5:4:2:1). To visualize the composition of EPS, the gel was immersed in aniline-diphenylamine reagent and baked in an oven at 110 °C for 5 minutes. To perform Benedict's test, polysaccharide and polysaccharide hydrolyzate were mixed with the same amount of Benedict's reagent (BIOZOA Biological Supply, Seoul, Korea) and then heated in boiling water. A monosaccharide component of the polysaccharide was determined by thin layer chromatography (TLC). The polysaccharide hydrolyzate was expressed at the same point as the standard substance glucose (FIG. 1B, Example 5). As a result of Benedict's test, the polysaccharide hydrolyzate changed the color of the reagent to orange-red. Further, the polysaccharide changed the color of the reagent to green (FIG. 1C, Example 5). Through these results, it can be seen that the separated polysaccharide is mainly composed of glucose.

### 3. Fourier transform infrared spectroscopy (FTIR) and gel permeation chromatography (GPC)

Structural characteristics of polysaccharides were analyzed using TENSOR27 FTIR (Bruker, Billerica, MA, USA) in the absorption range of 4000-500 cm⁻¹ at Seoul National University's National Intercollegiate Research Facility Center. Further, GPC analysis was performed using a Dionex HPLC Ultimate 3000 RI System (Thermo Scientific, Waltham, MA, USA) with 120 Å, 500 Å, and 1000 Å columns (Waters, Milford, MA, USA) at 40 °C to determine molecular weights of the polysaccharides. Experimental data were corrected with pullulan and processed with a chromatography data system (Chromeleon 6.8 Extension-pak). The polysaccharides were eluted with 0.1 M sodium azide in water and operated at a flow rate of 1 mL/min.

FTIR results of the polysaccharide of Example 5 showed a complex peak pattern from 3500 cm⁻¹ to 500 cm⁻¹. Among them, the 3307.31 cm⁻¹ peak represents an OH group, the 2935.1 cm⁻¹ peak represents a weak CH stretch peak of a methyl group, and the 1648.88 cm⁻¹ peak represents a characteristic group of glucose such as a C=O stretch peak. Further, the 1032.58 cm⁻¹ peak, which is the strongest absorption band, shows C-O and O-H bonds. The 911.98 cm⁻¹ and 812.28 cm⁻¹ peaks represent the flanking groups of carbohydrates (FIG. 1D, Example 5). From these results, it was confirmed that the separated polysaccharide had an absorption peak of polysaccharide containing glucose as a main component. In addition, as a result of molecular weight calculation through GPC, the polysaccharide of Example 5 had a number average molecular weight (Mn) of 1.84 × 10⁴ Da, a weight average molecular weight (Mw) of 7.57 × 10⁴ Da, and a size average molecular weight (Mz) of 3.74 × 10⁵ Da, and a polydispersity index (PDI) of 4.12 (FIG. 1E). Through these results, it was confirmed that the *Lactobacillus plantarum*-derived polysaccharide was a homogeneous polysaccharide mainly composed of glucose.

### IV. Confirmation of anti-inflammatory efficacy of Lactobacillus plantarum extract or Lactobacillus plantarum-derived polysaccharide

### 1. Experimental method

### (1) Cell culture, materials and statistics

Mouse macrophage line RAW 264.7 was obtained from the American Type Culture Collection. Cells were incubated with Dulbecco's modified Eagle's media (WELGENE, Daegu, Korea) including 10% fetal bovine serum (HyClone, Logan, UT, USA) and 1% penicillin/streptomycin (Gibco, Grand Island, NY, USA) in an incubator in a humidified 5% CO₂ atmosphere at 37 °C conditions. Antibodies were purchased from the following sources: phospho-NF-κB, NF-κB, phospho-ERK, ERK, phospho-p38, p38, phospho-JNK, JNK, and COX-2 were purchased from Cell Signaling Technology (Danvers, MA, USA); HO-1 was purchased from Abcam (Cambridge, UK); NRF2 and TLR4 were purchased from Cusabio (Wuhan, China); iNOS was purchased from Invitrogen (Carlsbad, CA, USA); MyD88 was purchased from Novus Biologicals (Centennial, CO, USA); and GAPDH was purchased from BioLegend (San Diego, CA, USA). All data were obtained from three independent experiments and were expressed as mean ± standard deviation (SD). Statistical analysis was determined using unpaired ANOVA, and significance was defined as *p<0.05, **p<0.01, ***p<0.001.

### (2) Cell viability assay

To determine an effect on cell viability of the *Lactobacillus plantarum* L-14 strain, RAW 264.7 cells were seeded on 96-well plates at a density of 2.0 × 10³ cells per well. After one day, the medium was replaced with a medium containing the L-14 strain and cultured for an additional 6 hours. Then, the medium containing the L-14 strain was replaced with a fresh medium containing LPS to induce an inflammatory response at the indicated concentration for 6 hours. Cell viability was confirmed using the Quanti-Max^{™} WST-8 cell viability kit (BIOMAX, Seoul, Korea).

Further, in order to confirm the effect of L-14-derived EPS (Example 5) on cell viability, the inoculated cells were cultured for 1 day in a medium containing various concentrations of L-14-derived EPS (Example 5). Viability was confirmed with the same kit.

### (3) ELISA

RAW264.7 cells were seeded on 12-well plates at a density of 2.0 × 10⁵ cells per well. Then, the medium was replaced with a medium inoculated with the L-14 strain at a concentration of 1.0 × 10⁶ CFU/mL and maintained for 6 hours. Thereafter, the medium was removed, and the cells were thoroughly washed three times with DMEM. To induce inflammation, the washed cells were cultured in a medium containing LPS (1 g/mL) and maintained for 6 hours. The culture solution obtained from each well was centrifuged at 10,000 g for 3 minutes, and the supernatant was collected. Cytokines were quantified by the ELISAMAX-Deluxe Set (BioLegend) according to the manufacturer's recommendations.

Further, in order to investigate whether pretreatment with L-14-derived EPS (Example 5) reduces the induction of cytokines by LPS, RAW264.7 cells were inoculated into 12-well plates for 24 hours. After the cells were pretreated with L-14-derived EPS (Example 5) for 6 hours, the cultured medium was replaced with a fresh medium containing LPS for 18 hours to induce an inflammatory response. Cytokines in the culture medium were quantified as described above.

### (4) Crystal violet dyeing

RAW264.7 cells were seeded on 12-well plates and cultured for 1 day. To investigate whether EPS pretreatment affects the morphology of cells and inhibits morphological changes induced by LPS, cells were treated with EPS (Example 5) for 6 hours and the culture medium was replaced with a fresh medium containing LPS (1 g/mL) for 18 hours. Then, cells were washed with PBS and stained with a crystal violet solution (Sigma-Aldrich, Saint Louis, MO, USA). Morphological changes were measured at 100 magnification using an EVOS CL Core microscope (Life Technologies, Carlsbad, CA, USA).

### (5) Western blot

Proteins were isolated from LPS-treated RAW 264.7 cells using Cell Culture Lysis 1 × Reagent (Promega, Fitchburg, WI, USA) along with a protease inhibitor cocktail and a phosphatase inhibitor cocktail. Cytoplasm and nuclear proteins were obtained using the ExKine-Nuclear and Cytoplasmic Protein Extraction Kit (Abbkine, Wuhan, China) according to the manufacturer's instructions. A total protein concentration was quantified with a Pierce-BCA protein assay kit (Thermo Scientific, Waltham, MA, USA). The denatured protein was then separated by 12% sodium dodecyl sulfate-polyacrylamide gel electrophoresis, and transferred to a polyvinylidene difluoride membrane. After blocking for 1 hour at room temperature (RT), the membranes were incubated overnight at 4 °C in skim milk containing an appropriate primary antibody (1:1000). The membrane was washed using 0.1% Tris buffered saline. It was further incubated for 1 hour at room temperature in skim milk containing Tween 20 (Sigma) and a secondary antibody (1:2000). Protein signals were detected using ECL Western Blot Substrate (Daeil Lab Service, Seoul, Korea).

### (6) Immunofluorescence (IF) analysis

RAW 264.7 cells were seeded on 6-well plates at a density of 1.0 × 10⁶ cells per well and incubated overnight. The cells pretreated with EPS (Example 5) were cultured in a fresh medium containing LPS and harvested by scraping. After fixation with 4% paraformaldehyde and permeabilization with 0.1% Triton X-100 (Sigma), the cells were blocked with 3% bovine serum albumin (BSA, Bovogen, East Keilor, Australia) for 1 hour. Then, these were incubated along with PE-conjugated iNOS antibody. The washed cells were mounted on ProLong-Glass Antifade Mountant (Invitrogen) containing NucBlue-Stain. Further, in order to visualize the translocation of NF-κB, RAW264.7 cells were pretreated with EPS for 2 hours and the inflammatory response was stimulated with LPS (1 g/mL) for 1 hour. Separated cells were prepared in the same manner. After blocking for 1 hour, the cells were incubated along with NF-B antibody (1:200) at 4 °C for 18 hours. Then, the cells were thoroughly washed and incubated along with Alexa Fluor 488-conjugated secondary antibody in 3% BSA at room temperature for 30 minutes. The cells were mounted using the same strain. All slides were analyzed using an LSM 800 confocal laser scanning microscope 293 (Carl Zeiss, Oberkochen, Germany).

### 2. Experimental results

### (1) Confirmation of inflammatory response inhibitory efficacy of metabolites secreted from Lactobacillus plantarum strain by LPS in mouse macrophages

Inflammatory response inhibitory efficacy of *Lactobacillus plantarum* L-14 strain (KTCT13497BP) was confirmed by quantifying LPS-induced cytokines in mouse macrophage RAW264.7 (American Type Culture Collection) through enzyme-linked immunosorbent assay (ELISA). Cells seeded on 12-well plates were co-cultured along with L-14 strain for 6 hours, and IL-6, TNF-α and monocyte chemoattractant protein-1 (MCP-1), which are inflammatory markers, were induced by LPS treatment. As a result, the expression levels of IL-6, TNF-α and MCP-1 were increased by LPS treatment, but were not increased by L-14 treatment alone. The release of inflammatory cytokines was significantly reduced in the cells co-cultured with L-14 as compared to the control group (LPS-treated group) (FIG. 2A). Further, in order to confirm whether the L-14 strain affects cell proliferation to cause a reduction in the inflammatory cytokines, the viability of RAW 264.7 cells cultured under the same culture conditions was quantified. As a result, cell viability was not affected by L-14 or LPS (FIG. 2B). These results suggest that metabolites secreted from *Lactobacillus plantarum* L-14 directly interact with immune cells to exhibit anti-inflammatory effects.

### (2) Confirmation of morphological change inhibitory efficacy of Lactobacillus plantarum-derived EPS by LPS in mouse macrophages

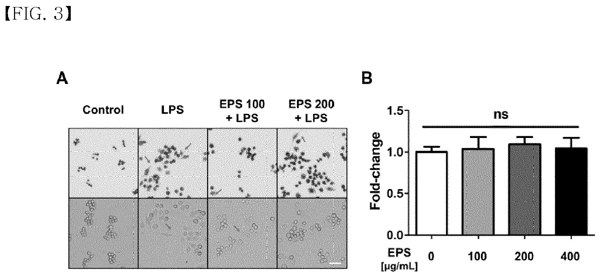

To determine whether *Lactobacillus plantarum*-derived EPS inhibits morphological changes induced by LPS, mouse macrophages were pretreated with EPS of Example 5 for 6 hours, followed by stimulating morphological changes with LPS for 18 hours, to confirm the form of the cells. As a result of crystal violet staining, L-14-derived EPS (Example 5) alleviated the morphological deformation of the cells caused by LPS treatment (FIG. 3A, EPS100 in FIG. 3 means 100 ug/ml treatment in Example 5 and EPS200 means 200 ug/ml treatment in Example 5). Further, cell viability was maintained unaffected when RAW264.7 cells were treated with higher concentrations of EPS for 1 day (FIG. 3B). These results indicate that *Lactobacillus plantarum*-derived EPS inhibited morphological changes induced by LPS in mouse macrophages without affecting cell viability.

### (3) Confirmation of inflammatory response inhibitory efficacy of Lactobacillus plantarum-derived EPS by LPS in mouse macrophages

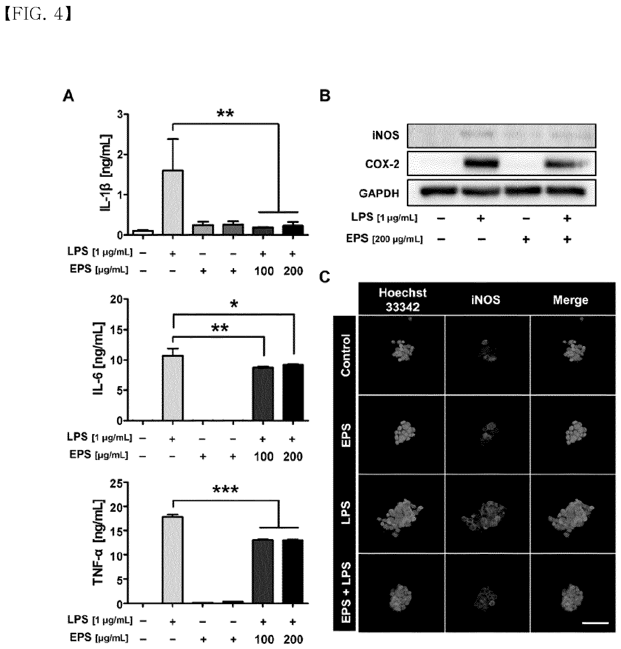

To determine whether *Lactobacillus plantarum*-derived EPS inhibits the inflammatory response caused by LPS stimulation, pro-inflammatory cytokines generated in RAW 264.7 cells pretreated with EPS of Example 5 were quantified. As a result of cytokine quantification, pretreatment using L-14-derived EPS has attenuated the levels of IL-6, TNF-α and IL-1β, and in particular, IL-1β was reduced similarly to the expression level of the control group (FIG. 4A, EPS in FIG. 4 means Example 5). Further, expression levels of COX-2 and inducible nitric oxide synthase (iNOS), known as major mediators of inflammation, were analyzed in RAW 264.7 cells pretreated with EPS of Example 5 through Western blot. Although the expression levels of COX-2 and iNOS proteins increased after LPS treatment, these expression levels were inhibited in EPS-pretreated RAW 264.7 cells (FIG. 4B). Further, the expression level of LPS-induced iNOS was analyzed in RAW 264.7 cells pretreated with EPS of Example 5 through immunofluorescence (IF) analysis. As a result of the analysis, the expression of LPS-induced iNOS was increased after LPS treatment but the expression of LPS-induced iNOS was decreased in EPS-pretreated RAW 264.7 cells (FIG. 4C). Through these results, it was confirmed that the *Lactobacillus plantarum*-derived EPS exhibits an inhibitory effect on the LPS-induced inflammatory response.

### (4) Confirmation of NF-κB nuclear translocation inhibitory efficacy of Lactobacillus plantarum-derived EPS by LPS in mouse macrophages

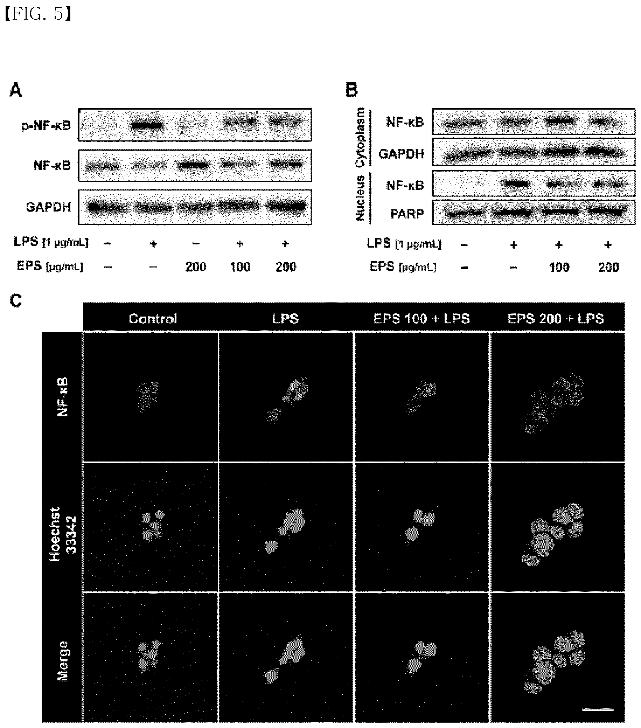

To determine whether *Lactobacillus plantarum*-derived EPS inhibits LPS-induced nuclear translocation and phosphorylation of NF-κB, after inducing an inflammatory response with LPS in RAW 264.7 cells pretreated with EPS of Example 5, the expression level of NF-κB and the location of the phosphorylated form were analyzed. As a result, a ratio of p-NF-κB/NF-κB was decreased by pretreatment with L-14-derived EPS (Example 5) (FIG. 5A, EPS in FIG 5 means Example 5). L-14-derived EPS (Example 5) itself did not promote phosphorylation of NF-κB. On the other hand, L-14-derived EPS (Example 5) inhibited the LPS-induced nuclear translocation of NF-κB at all concentrations (FIG. 5B). Consistently, the nuclear translocation of NF-κB was induced by LPS, but was reduced by pretreatment with L-14 derived EPS (Example 5) (FIG. 5C, EPS100 in FIG. 5C means 100 ug/ml treatment in Example 5 and EPS200 means 200 ug/ml treatment in Example 5).

### (5) Identification of inflammation inhibition pathway of Lactobacillus plantarum-derived EPS in mouse macrophages

### 1) Regulation of mitogen-activated protein kinase (MAPK) and nuclear factor E2-related factor 2/heme oxygenase-1 (NRF2/HO-1) pathways

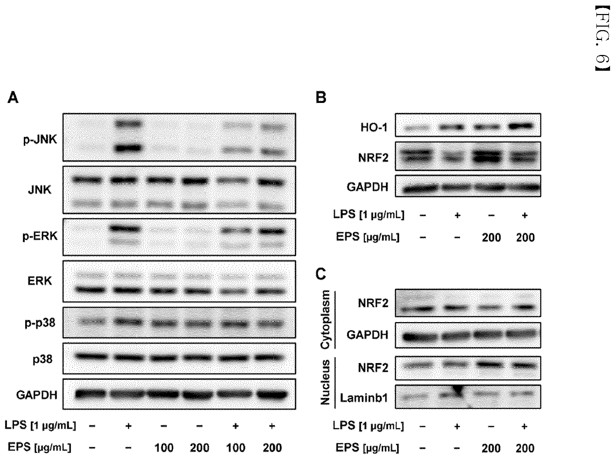

The MAPK and NRF2/HO-1 pathways are known to be key regulators of the inflammatory response in mouse macrophages. First, in order to determine whether *Lactobacillus plantarum*-derived EPS can inhibit the MAPK pathway in LPS-induced RAW264.7 cells, phosphorylation of MAPK family proteins (JNK, ERK and p38) was analyzed by Western blot. As a result, the EPS of Example 5 significantly inhibited the phosphorylation of JNK and ERK even at a concentration of 100 g/mL (FIG. 6A, EPS in FIG. 6 means Example 5). The phosphorylation of p38 was inhibited when the EPS of Example 5 was treated at a concentration of 200 g/mL. Further, in order to determine whether the anti-inflammatory effect of *Lactobacillus plantarum*-derived EPS is mediated through the NRF2/HO-1 pathway, protein expression levels of NRF2 and HO-1 markers were investigated. As a result, the expression levels of HO-1 and NFR2 increased with or without LPS (FIG. 6B). Further, the EPS of Example 5 increased the nuclear translocation of NRF2, which is known as a major pathway mediating the inflammatory response (FIG. 6C). That is, *Lactobacillus plantarum*-derived EPS inhibited the phosphorylation of MAPK family proteins and improved the expression of NRF2/HO-1 in LPS-induced RAW 264.7 cells.

### 2) Inhibition of interaction between LPS and TLR4

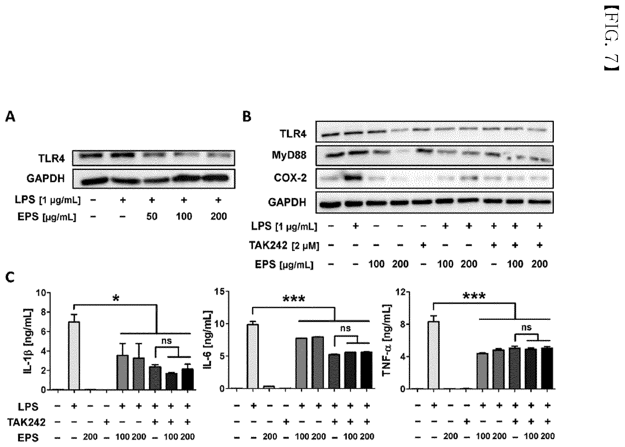

To find out whether *Lactobacillus plantarum*-derived EPS inhibits the inflammatory response through TLR4, the expression level of TLR4 was analyzed in RAW264.7 cells after treatment with EPS and LPS of Example 5. As a result, TLR4 up-regulated by LPS was reduced by EPS (at all concentrations) (FIG. 7A, EPS in FIG. 7 means Example 5). Further, it was confirmed how *Lactobacillus plantarum-*derived EPS interacts with TLR4 using TAK-242, which was confirmed to block the TLR4 pathway. Interestingly, when RAW264.7 cells were treated with EPS only, the protein expression of TLR4 was more inhibited than that of the untreated control group (FIG. 7B). EPS also inhibited the expression levels of TLR4 and MyD88 in the LPS-induced group, similar to that observed in the TAK-242 treatment group. The expression of COX-2 was inhibited by TAK-242, and was also inhibited by EPS in the same way. Consistently, EPS significantly reduced the expression of the cytokines IL-1, IL-6 and TNF- secreted in the medium to the extent that TAK-242 down-regulated them (FIG. 7C). The above results suggest that *Lactobacillus plantarum*-derived EPS exhibits anti-inflammatory effects through TLR4 in LPS-treated RAW 264.7 cells.

### V. Confirmation of anti-obesity, insulin resistance, and fatty liver inhibitory efficacy of Lactobacillus plantarum extract or Lactobacillus plantarum-derived polysaccharide

### 1. Experimental method

### (1) Cell culture, materials and statistics

3T3-L1 cell line and hBM-MSC were obtained from the American Type Culture Collection (Manassas, VA, USA) and PromoCell (Heidelberg, Germany), respectively. 3T3-L1 cells were cultured in Dulbecco's modified Eagle's medium (DMEM; GE Healthcare, Chicago, IL, USA) containing 4.5 g/L D-glucose, 10% fetal bovine serum (FBS), 1% penicillin/streptomycin (P/S), 25 mM HEPES, 3.7 g/L sodium bicarbonate, 4 mM L-glutamine and 1 mM sodium pyruvate. The 3T3-L1 cells were cultured at 37°C in an incubator including a humidified atmosphere of 5% CO2.

Rodent Diet containing 60% kcal fat was purchased from Research Diets (New Brunswick, NJ, USA). In addition, AICAR and CC were purchased from Selleckchem (Houston, TX, USA), and C29 was purchased from Cayman Chemical (Ann Arbor, MI, USA). Insulin, leptin, adiponectin and resistin ELISA kits were purchased from CUSABIO (Hubei, China), and IFN-γ, IL-6 and MCP1 ELISA kits were purchased from BioLegend (San Diego, CA, USA). Antibodies were purchased from the following sources: Akt, rabbit IgG isotype and goat IgG isotype antibodies were purchased from Bioss (Woburn, MA, USA); PPARγ, C/EBPα, FABP4, t-AMPKα, p-AMPKα, t-ACC, p-ACC, FAS, p-NF-κB, t-NF-κB, p-AKT, t-AKT, t-AS160, p-AS160 and MyD88 antibodies were prepared from Cell Signaling Technology (Danvers, MA, USA); Arg1, ATGL, IL-6, TNF-α and TLR2 antibodies were purchased from CUSABIO; SREBP-1c antibody was purchased from Novus Biologicals (Centennial, CO, USA); leptin and resistin antibodies were purchased from R&D Systems (Minneapolis, MN, USA); and β-actin, GAPDH and SCD1 antibodies were purchased from Santa Cruz Biotechnology (Dallas, TX, USA).

Statistical analysis was performed using SPSS software (version 25.0; SPSS Inc., Chicago, Illinois) and GraphPad Prism 5 (GraphPad, CA, USA). Data are expressed as mean ± standard deviation. Statistical analysis was determined using ANOVA, and significance was defined as *p<0.05, **p<0.01, ***p<0.001.

### (2) Differentiation of 3T3-L1 cells and hBM-MSCs; and fat accumulation and triacylglycerol analysis

3T3-L1 cells or hBM-MSCs were seeded on 24-well plates in a medium at a density of 1.0 × 10⁵ cells per well. Two days after the cells were fused, the medium was replaced with an adipogenic induction medium (MDI) containing alpha-MEM, 10% FBS, 1% P/S, 1 uM dexamethasone, 0.5 mM isobutylmethylxanthine, 100 uM indomethacin, 10 mg/mL insulin, *Lactobacillus plantarum* extract (Examples 1 to 4), *Lactobacillus plantarum* extract with adjusted pH 7.0 (P60), and *Lactobacillus plantarum* extract incubated at 90 °C for 30 minutes. 4 days after the first adipogenesis induction, the medium was replaced with an adipogenic maintenance medium containing alpha-MEM, 10% FBS, 1% penicillin/streptomycin, 10 mg/mL insulin and the extract. The medium was replaced with a new one every 2 days during adipogenic differentiation, and 3T3-L1 cells and hBM-MSCs were maintained for 12 and 7 days, respectively. In order to compare lipid accumulation after 12 days, 3T3-L1 cells and hBM-MSCs were washed with PBS, fixed with 4% formaldehyde, and stained with Oil red O solution for 30 minutes. The stained adipocytes were observed at 200 times magnification by an EVOS CL Core microscope (Life Technologies). In order to compare relative lipid accumulation, Oil red O of 3T3-L1 cells and hBM-MSCs was dissolved in isopropanol and quantified by measuring absorbance at 500 nm using a microplate reader. The results were analyzed as a percentage with respect to the control value which was set to be 1.0. The formula to indicate relative lipid accumulation was (Asample - Ablank)/(Acontrol - Ablank). In addition, triacylglycerol (TAG) was quantified with a TAG assay kit (Cayman Chemical) according to the manufacturer's instructions.

### (3) Cell viability assay

3T3-L1 cells were seeded on 96-well plates at a density of 1.0 × 10³ cells per well. After 24 hours, the medium was replaced with various concentrations of *Lactobacillus plantarum* extract (Examples 1 to 4) and maintained for 4 days. Cell viability was confirmed with the WST-1 cell viability assay kit (Dongin LS, Seoul, Korea) .

### (4) Western blot analysis

3T3-L1 cells and hBM-MSCs were harvested according to conditions, and lysed in Cell Culture Lysis 1X Reagent (Promega) containing a mixture of protease and phosphatase inhibitor (MCE) on ice for 5 minutes. Insoluble debris was removed by centrifugation at 15,000 g for 15 minutes at 4 °C. Totally 10-40 µg of protein was isolated from the separated supernatant using 8-12% SDS-PAGE, and transferred to a polyvinylidene difluoride membrane. The membrane was incubated in 0.1% Tween 20 Tris-buffered saline (TBST) containing 5% bovine serum albumin (BSA) for 1 hour at room temperature. The membrane was incubated overnight in 5% BSA-TBST along with the primary antibody at 4 °C. After washing three times with TBST, the membrane was incubated in 5% BSA-TBST conjugated with horseradish peroxidase at room temperature for 1 hour. Membrane protein signals were developed and analyzed in ECL Western Blotting Substrate (DAEILLAB SERVICE). All experiments were repeated three times.

### (5) Real-time quantitative PCR

mRNA was isolated from 3T3-L1 cells and incubated with *Lactobacillus plantarum* extracts (Examples 1 to 4) using PureLink^{™} RNA Mini Kit (Invitrogen, Carlsbad, CA, USA), followed by reverse-transcription into cDNA using cDNA kit (Promega, Madison, WI, US). Then, cDNA was amplified and analyzed with TB Green Mix (TAKARA, Shinga, Japan) using StepOnePlus^{™} Real-Time PCR System (Applied Biosystems, Foster City CA, USA). The primers used for RT-qPCR are shown in Table 1 below.

**[TABLE 1]**

| Divisi on | Gene | 5' -> 3' | Sequence | SEQ ID NO. |
|---|---|---|---|---|
| Mouse primer | PPARγ | Forward | TTCAGAAGTGCCTTGCTGTG | 1 |
| | | Reverse | GCTGGTCGATATCACTGGAGA | 2 |
| | C/EBPα | Forward | GGTGCGTCTAAGATGAGGGA | 3 |
| | | Reverse | CCCCCTACTCGGTAGGAAAA | 4 |
| | FABP4 | Forward | AAGGTGAAGAGCATCATAACCCT | 5 |
| | | Reverse | TCACGCCTTTCATAACACATTCC | 6 |
| | LPL | Forward | ATGGATGGACGGTAACGGGAA | 7 |
| | | Reverse | CCCGATACAACCAGTCTACTACA | 8 |
| | FAS | Forward | ATCCGGAACGAGAACACGATCT | 9 |
| | | Reverse | AGAGACGTGTCACTCCTGGACTT | 10 |
| | GPDH | Forward | ATGGCTGGCAAGAAAGTCTG | 11 |
| | | Reverse | CGTGCTGAGTGTTGATGATCT | 12 |
| | CD36 | Forward | AGATGACGTGGCAAAGAACAG | 13 |
| | | Reverse | CCTTGGCTAGATAACGAACTCTG | 14 |
| | GAPDH | Forward | AGGTCGGTGTGAACGGATTTG | 15 |
| | | Reverse | TGTAGACCATGTAGTTGAGGTCA | 16 |
| Human primer | PPARγ | Forward | ACCAAAGTGCAATCAAAGTGGA | 17 |
| | | Reverse | ATGAGGGAGTTGGAAGGCTCT | 18 |
| | C/EBPα | Forward | AACACGAAGCACGATCAGTCC | 19 |
| | | Reverse | CTCATTTTGGCAAGTATCCGA | 20 |
| | FABP4 | Forward | ACTGGGCCAGGAATTTGACG | 21 |
| | | Reverse | CTCGTGGAAGTGACGCCTT | 22 |
| | Leptin | Forward | TGCCTTCCAGAAACGTGATCC | 23 |
| | | Reverse | CTCTGTGGAGTAGCCTGAAGC | 24 |
| | GPDH | Forward | CTATACAGCATCCTCCAGCACAA | 25 |
| | | Reverse | GGCCCTCGTAGCACACCTT | 26 |
| | CD36 | Forward | CTTTGGCTTAATGAGACTGGGAC | 27 |
| | | Reverse | GCAACAAACATCACCACACCA | 28 |
| | GAPDH | Forward | TGGACTCCACGACGTACTCA | 29 |
| | | Reverse | ACATGTTCCAATATGATTCC | 30 |

### (6) Animals, diet and study design

Animal studies were performed at an accredited animal facility center in the College of Dentistry, Seoul National University. Procedures on animals were carried out in accordance with the Korean Laws on animal testing, and the research was approved by the Animal Management Committee of Seoul National University (SNU-180309-1).

Four-week-old C57BL/6J male mice were purchased from Orient Bio (Seongnam, Korea). Mice were randomly divided into three groups: (i) a normal diet (ND, n=6); (ii) a high fat diet (HFD) (n=7); and (iii) a high fat diet (HFD) treated with L-14 diet (L-14 extract (Example 1)) (n=8). The mice were fed ND or HFD for an additional 7 weeks, and the mice had free access to water. L-14 extracts (500 mg/kg body weight) were orally administered by a needle catheter every 2 days for the duration of the animal study to the L-14 diet group. In order to give the same stress to the other two groups, PBS was orally administered under the same conditions. Body weight and food intake were measured every other day. After a feeding and administration period of 7 weeks, the mice were fasted overnight and euthanized. White adipose tissues of the epidermis and inguinal were collected and weighed. Liver and serum were immediately isolated for further study. Total protein was isolated from mouse adipose tissue using SuperFastPrep-2^{™} (MP Biomedicals, Irvine, CA, USA), and Western blot analysis was performed. Serum biochemical analysis was performed at the Korea Mouse Phenotyping Center (Seoul, Korea), and hormones and cytokines in mouse serum were quantified using an ELISA kit according to the manufacturer's recommended manual.

### (7) Mouse epidermal white adipose tissue (eWAT) and liver histology

Mouse eWAT and liver were rinsed twice with sterile PBS and fixed overnight in 4% paraformaldehyde in PBS. Tissues were cut into 5 um pieces, inserted into paraffin blocks, then placed on an adhesion microscope (Paul Marienfeld, Lauda-Konigshofen, Germany). Wax was removed and the rehydrated portion was stained with hematoxylin and eosin. For immunohistochemistry, antigens in sections were recovered for 10 minutes in a pressure vessel filled with citrate buffer (pH 7.0). Sections were immersed in BLOXALL^{®} Endogenous Peroxidase Solution (Vector Laboratories, Burlingame, CA, USA) for 20 minutes at room temperature and incubated in 2.5% normal horse serum to reduce nonspecific binding. The antibody was diluted 1:100-200 with 2.5% normal horse serum and the sections were incubated overnight at 4°C along with the diluted primary antibody. Rabbit IgG antibody and goat IgG antibody were used as negative controls. Then, the sections were incubated along with ImmPRESS polymer anti-rabbit IgG reagent and anti-goat IgG reagent for 30 minutes at room temperature. The sections were stained with ImmPACT^{®} DAB Peroxidase (HRP) substrate (Vector Laboratories) and then lightly counterstained with hematoxylin. Images were obtained with a microscope (BX50, Olympus, Tokyo, Japan).

### (8) Analysis of the effects of Lactobacillus plantarum extract and Lactobacillus plantarum-derived polysaccharide on AMPK and TLR2 signaling pathways

3T3-L1 cells were seeded on 24-well plates in a medium at a density of 1.0 × 10 ⁵ cells per well. Two days after the cells were fused, the medium was replaced with starvation medium containing only DMEM and 1% P/S for 1 hour. After starvation, the medium was replaced with a normal medium containing 250 µM 5-aminoimidazole-4-carboxamide ribonucleotide (AICAR), 5 µM Compound C (CC) and 50 µM C29, and the cells were cultured for 2 hours. The cells were induced to differentiate into mature adipocytes in MDI with L-14 extract (Example 1) or *Lactobacillus plantarum*-derived polysaccharides (Examples 6 to 9). AICAR and C29 were treated every 2 days and CC was not further treated for the entire period. After 12 days, the adipogenesis inhibitory effect of L-14 extract or *Lactobacillus plantarum*-derived polysaccharide through AMPK and TLR2 signaling pathways was analyzed by Oil red O staining and TAG analysis. Further, in order to investigate the effect of L-14 extract or *Lactobacillus plantarum-*derived polysaccharide in the initial stage of adipogenic differentiation, protein was isolated on the 4th day and analyzed by Western blot analysis.

### 2. Experimental results

### (1) Confirmation of the efficacy of Lactobacillus plantarum extract to inhibit differentiation of 3T3-L1 preadipocytes and hBM-MSC into mature adipocytes

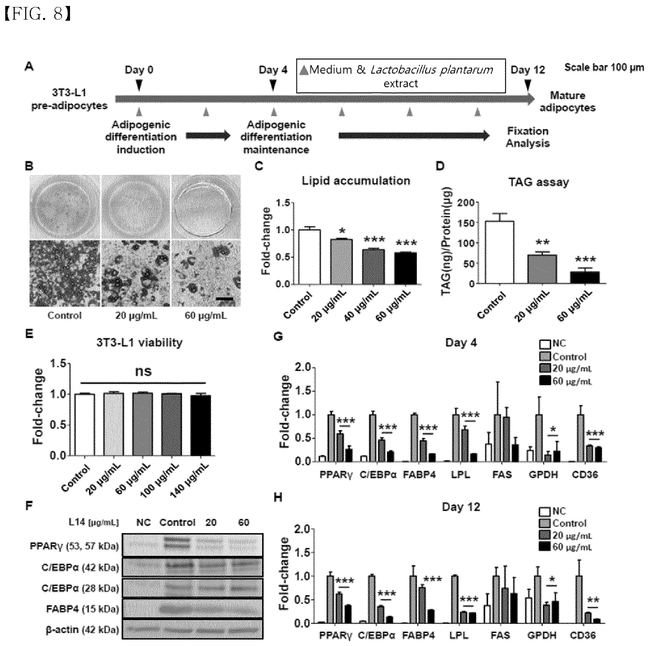

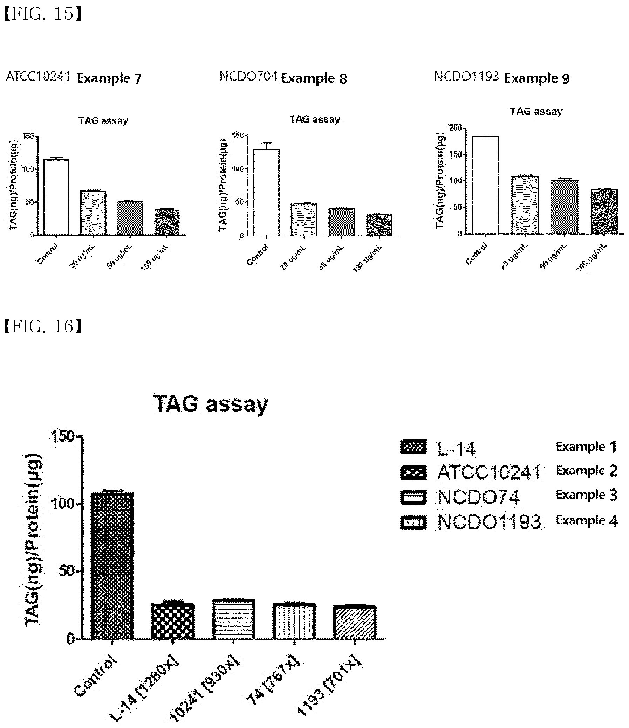

The following experiment was performed to confirm whether the *Lactobacillus plantarum* extract exhibits adipogenesis inhibitory effect. 3T3-L1 cells were treated with *Lactobacillus plantarum* extracts (Examples 1 to 4) at concentrations of 20 and 60 ug/mL every 2 days in the course of adipogenic differentiation (FIG. 8A). As a result of Oil red O staining, lipid accumulation was dose-dependently inhibited by L-14 extract (FIGS. 8B and 8C, scale bar = 100 µm, FIGS. 8B and 8C show the experimental results for Example 1). Further, it was confirmed that the *Lactobacillus plantarum* extract (Examples 1 to 4) inhibited TAG storage through TAG assay (FIGS. 8D and 16, FIGS. 8D and 16 show the experimental results in Examples 1 and 2 to 4 in order, respectively). Amounts of L-14 (32.015 mg), ACTT10241 (23.247 mg), NCDO71 (19.181 mg), and NCDO1193 (17.532 mg) were obtained on the bases of the cell protein content extracted based on the 1L culture solution of each lactic acid strain, respectively. Further, based on the same culture volume, L-14 could be obtained with a high cell at the fastest growth rate. When the same amount of protein was treated based on the protein quantified from the cell extract, it was confirmed that a reduction effect of about 75% compared to the control group was exhibited. That is, the amount required to show a reduction effect of about 75% is L-14 (1280-fold dilution_1280x) and ACTT10241 (930-fold dilution_930x), NCDO71 (767-fold dilution_767x), and NCDO1193 (701-fold dilution_701x), in terms of dilution magnification based on protein of 1L cultured cell (corresponding to 25 ug/ml concentration). Through these results, it could be seen that among the *Lactobacillus plantarum* strains, the L-14 spawn could produce the most anti-obesity substances. The L-14 extract (Example 1) did not affect the cell viability of 3T3-L1 cells (FIG. 8E). This means that the inhibitory effect on adipogenic differentiation is not due to the cytotoxicity of the L-14 extract (Example 1). In addition, it was found through Western blot analysis that the L-14 extract (Example 1) caused a reduction in the protein expressions of peroxisome proliferator-activated receptor γ (PPARγ), CCAAT-enhancer-binding proteins α (C/EBPa), and fatty acid -binding protein 4 (FABP4), which are known as major adipogenesis markers (FIG. 8F). Further, as a result of confirming through RT-qPCR, consistent with the Western blot results, the L-14 extract (Example 1) caused a significant reduction in the gene expression of adipogenesis differentiation markers including PPARγ, C/EBPa, FAB4, lipoprotein lipase (LPL), fatty acid synthase (FAS), glycerol-3-phosphate dehydrogenase (GPDH), and CD36 in 3T3-L1 cells (FIGS. 8G and 8H). These markers were already reduced by the L-14 extract (Example 1) in the early stages of adipogenesis differentiation (days 0-4). These results indicate that adipogenesis inhibitory due to *Lactobacillus plantarum* extract can occur by reducing the expression of adipogenic factors in the early stage of differentiation. In FIG. 8, NC refers to cells cultured in the normal medium as a negative control.

### (2) Confirmation of the efficacy of Lactobacillus plantarum extract to inhibit weight gain and expression of inflammatory markers in HFD mouse model

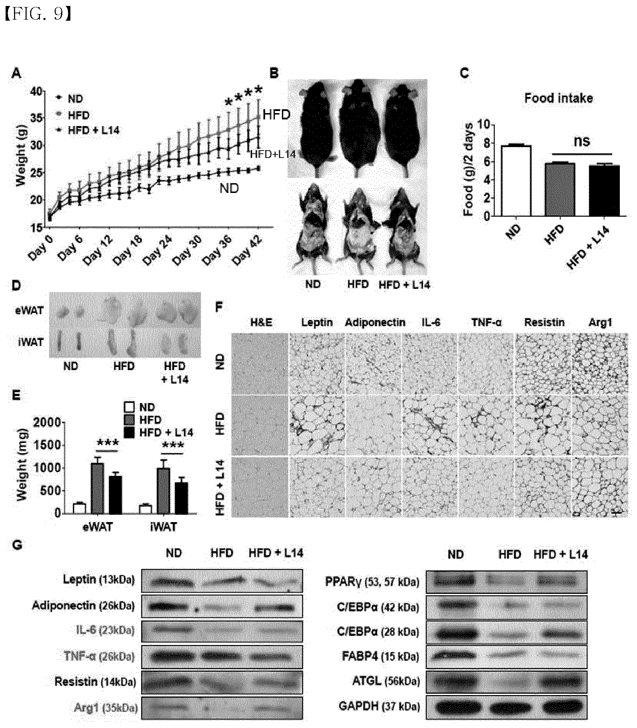

To determine the effect of *Lactobacillus plantarum* extract intake in vivo, C57BL/6J mice were randomly divided into three groups: a normal diet (n=6); a high fat diet (HFD) (n=7); and a high fat diet (HFD) treated with L-14 diet (L-14 extract (Example 1) (n=8). To the L-14 diet group, L-14 extract (Example 1) (500 mg/kg body weight) was orally administered by a needle catheter every 2 days for the duration of the animal study. In order to give the same stress to the other two groups, PBS was orally administered under the same conditions. As a result of oral administration, the average body weight of mice showed a significant difference after 36 days. In particular, the body weight of the L-14 diet group (31.51±1.96 g) showed a significant difference from the body weight of the HFD group (35.14±3.18 g) (FIGS. 9A and 9B, ND in FIG. 9 means the normal diet group, HFD means the high-fat diet group, HFD+L-14 means the L-14 diet group). There was no significant change in the food intake after oral administration of L-14 extract (FIG. 9C). Fat mass in epidermal white adipose tissue (eWAT) (1.10 ± 0.14 v.s. 0.81 ± 0.10 g) and inguinal white adipose tissue (iWAT) (0.99 ± 0.19 v.s 0.67 ± 0.12 g) in the L-14 diet group was significantly reduced as compared to the HFD group (FIGS. 9D and 9E). Through H&E staining, it was confirmed that the adipocyte size of eWAT was significantly smaller in the L-14 diet group than in the HFD group (FIG. 9F, scale bar = 100 µm). Further, the expression of inflammatory markers such as leptin, interleukin-6 (IL-6), tumor necrosis factor-α (TNF-α), and resistin was reduced in adipose tissue of the L-14 diet group, while the expression of anti-inflammatory markers such as adiponectin and arginase 1 (Arg1) was increased (FIGS. 9F and 9G).

### (3) Confirmation of the efficacy of Lactobacillus plantarum extract to inhibit insulin resistance marker and liver fat

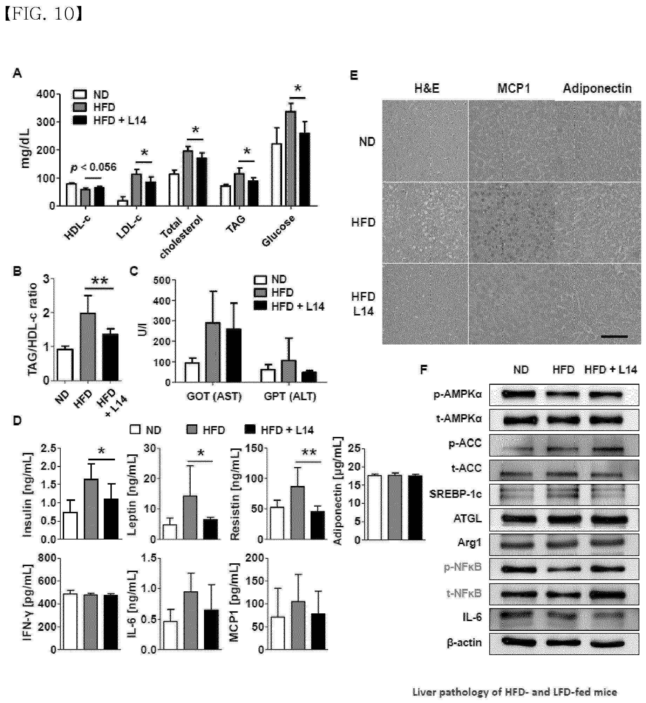

The effect of *Lactobacillus plantarum* extract for reducing insulin resistance marker and hepatic steatosis was confirmed through blood chemistry test and ELISA. According to blood chemistry test, low-density lipoprotein cholesterol (LDL-c) and TAG levels were significantly reduced in L-14 diet mice compared to the HFD group, and high-density lipoprotein cholesterol (HDL-c) levels increased in parallel (p<0.056). (In FIG. 10A, ND in FIG. 10 means the normal diet group, HFD means the high-fat diet group, and HFD+L-14 means the L-14 diet group). Consistently, it was confirmed that total cholesterol was reduced by the L-14 extract (Example 1), and the serum insulin resistance marker TAG/HDL-c ratio was also significantly reduced (FIG. 10B). Fasting blood glucose, fasting insulin, leptin, and resistin were decreased, whereas GOT (AST), GPT (ALT), adiponectin, interferon γ (IFN-γ), IL-6, and monocyte chemoattractant protein 1 (MCP1) were not significantly changed (FIGS. 10C and 10D). Further, in order to confirm the effect of the L-14 extract (Example 1) on the liver, the liver tissue sample isolated from the HFD model was histologically analyzed. As a result of H&E staining, it was confirmed that the L-14 extract (Example 1) attenuated HFD-induced hepatic steatosis (FIG. 10E, scale bar = 100 µm). Furthermore, total protein was isolated from mice and livers using SuperFastPrep-2^{™} (MP Biomedicals, Irvine, CA, USA), and Western blot analysis was performed. It was confirmed that the L-14 extract (Example 1) inhibited the expression of MCP1 and IL-6, while activating the AMPK signaling pathway. There was no significant change in the expression of Arg1 and ATGL (adipose triglyceride lipase) involved in lipolysis, but IL-6 expression in the liver was decreased by the L-14 extract (FIG. 10F). Like the TAG inhibitory effect described above, it is sufficiently predictable that these insulin resistance markers and liver fat inhibitory effects would also appear in Examples 2 to 4, which are the same *Lactobacillus plantarum* extracts as in Example 1.

### (4) Confirmation of adipogenesis inhibitory efficacy in the early stage of adipogenesis differentiation of Lactobacillus plantarum extract

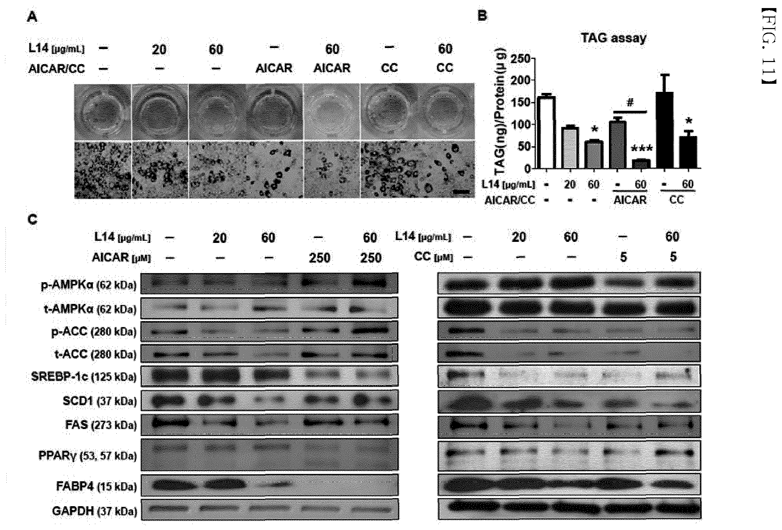

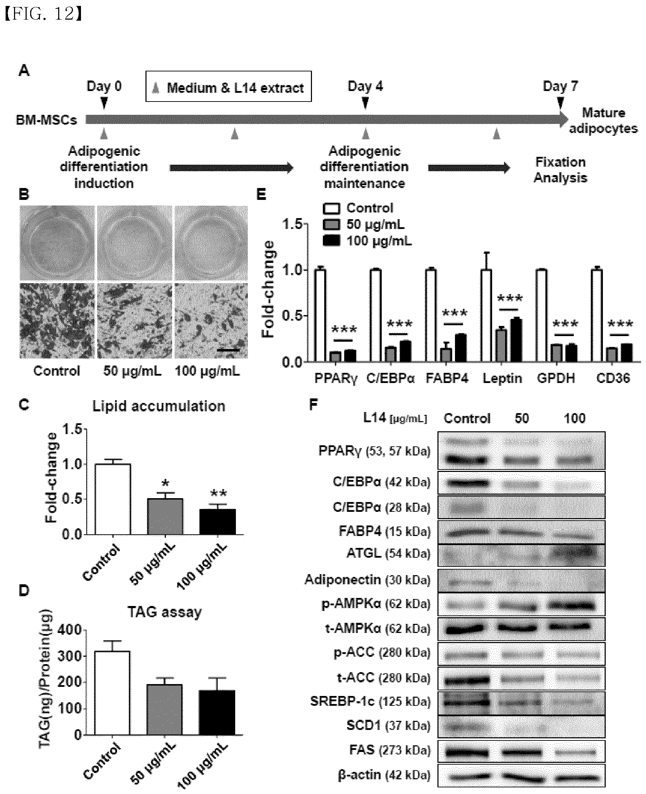

To determine whether the inhibitory effect of the *Lactobacillus plantarum* extract on adipogenesis differentiation is mediated by the AMPK signaling pathway, the L-14 extract (Example 1) together with the AMPK activator AICAR and the AMPK inhibitor CC 3T3-L1 cells were treated. As a result, AICAR significantly reduced the lipid content of 3T3-L1 cells after differentiation, and treatment with an additional L-14 extract (Example 1) improved the inhibitory effect of AICAR. CC did not affect lipid accumulation in 3T3-L1 cells, but the L-14 extract (Example 1) reduced TAG even under AMPK inhibition conditions (FIGS. 11A and 11B, scale bar = 100 um, L14 in FIG. 11 means Example 1). These results suggest that the anti- adipogenic effect of *Lactobacillus plantarum* extract is mediated by the AMPK signaling pathway. To determine whether the AMPK pathway could be activated in the early stages of adipogenic differentiation (days 0-4) by *Lactobacillus plantarum* extract, the markers were analyzed by Western blot. The AMPK pathway was synergistically activated by AICAR and L-14 extract (Example 1) within 4 days, and as a result, the expression of adipogenesis markers such as PPARγ and FABP4 was significantly reduced (FIG. 11C). Further, the L-14 extract (Example 1) increased the phosphorylation of AMPKα inhibited by CC. Simultaneous treatment of AICAR and L-14 extract (Example 1) inhibited TAG accumulation synergistically. Further, the L-14 extract (Example 1) inhibited the adipogenesis differentiation of human bone marrow mesenchymal stem cells (BM-MSC) by up-regulating the AMPK signaling pathway at the initial stage of differentiation (on the 4^{th} day) (FIG. 12, L14 in FIG. 12 means Example 1). Specifically, it was confirmed through Oil red O staining that the L-14 extract (Example 1) inhibited lipid accumulation in hBM-MSCs (scale bar = 100 um) (FIG. 12C). Also, it was confirmed that the L-14 extract (Example 1) inhibited TAG storage through TAG assay (FIG. 12D). The gene expression of adipogenic differentiation markers (PPARγ, C/EBPa, FABP4, leptin, GPDH and CD36) was also reduced by the L-14 extract (Example 1) (FIG. 12E). In hBM-MSC cultured with L-14 extract (Example 1) for 4 days, protein expression of major adipogenesis markers and adiponectin was decreased, and expression of AMPK signaling pathway marker was increased (FIG. 12F). These results suggest that *Lactobacillus plantarum* extract inhibits the differentiation of mouse preadipocytes and hBM-MSCs into mature adipocytes by up-regulating the AMPK signaling pathway in the early stage of adipogenic differentiation.

### (5) Confirmation of adipogenesis differentiation control efficacy of Lactobacillus plantarum-derived polysaccharide

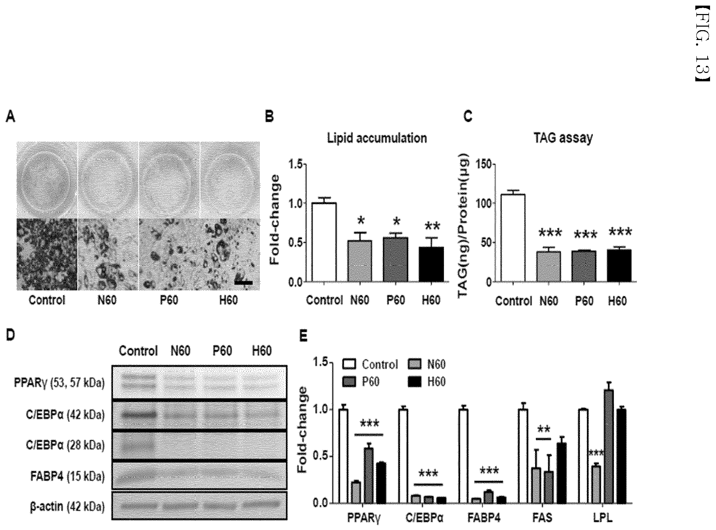

To determine what molecules can exhibit the adipogenesis inhibitory effect in the *Lactobacillus plantarum* extract, the adipogenesis inhibitory efficacy of the *Lactobacillus plantarum* extract (H60, P60) exposed to harsh temperature or pH conditions was confirmed. As a result, the adipogenesis inhibitory effect of the *Lactobacillus plantarum* extract was not changed by pH change or incubation under severe temperature conditions, which means that the effective molecule having the adipogenesis inhibitory effect was stable to heat (FIG. 13, scale bar = 100 µm).

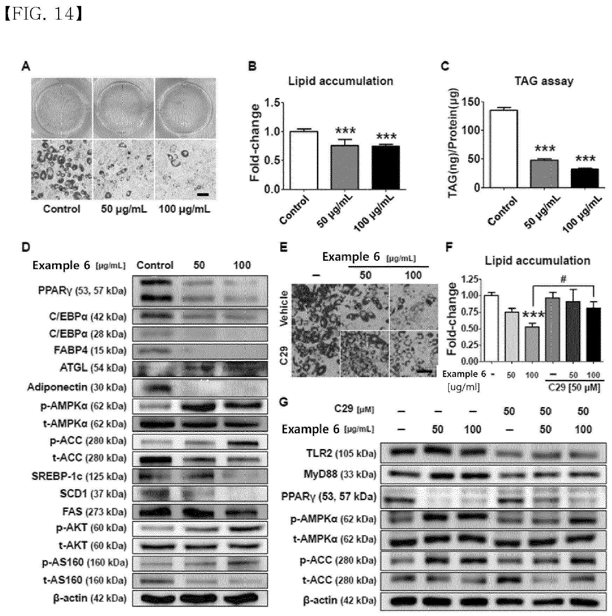

To determine whether the *Lactobacillus plantarum-*derived polysaccharide of Examples 5 to 9 can inhibit lipid accumulation, 3T3-L1 cells were cultured in MDI along with EPS for 12 days and analyzed. As a result of lipid accumulation analysis and TAG analysis, it was confirmed that all four types of *Lactobacillus plantarum* strain-derived polysaccharides had the adipogenesis inhibitory effect (FIGS. 14A to 14C and 15, FIG. 14 shows the experimental results of Example 6, and FIG. 15 shows the experimental results of Examples 7 to 9). Further, it was confirmed through Western blot analysis that *Lactobacillus plantarum*-derived polysaccharide up-regulated the AMPK pathway and down-regulated the expression of adipogenesis markers and adiponectin. Furthermore, it was confirmed that *Lactobacillus plantarum-derived* polysaccharide increased phosphorylation of AKT (protein kinase B) and 160 kDa Akt substrate (AS160), which are known to be related to intracellular glucose uptake (FIG. 14D). Additionally, further studies were performed using the TLR2 inhibitor C29 to determine which receptors in the cell interact with the *Lactobacillus plantarum*-derived polysaccharide. As a result, lipid accumulation was not affected by C29, but the adipogenesis inhibitory effect of *Lactobacillus plantarum-*derived polysaccharide was significantly reduced in the C29 treatment group (FIGS. 14E and 14F). Further, the activation of the AMPK pathway by EPS was inhibited in the early stage (on the 4^{th} day) when the expression of TLR2 and myeloid differentiation primary response 88 (MyD88) was inhibited by C29. This means that the *Lactobacillus plantarum*-derived polysaccharide interacts with TLR2 to activate the AMPK pathway, and consequently suppresses adipogenesis (FIG. 14G).

[The Deposit of Microorganism]
RECEIPTE IN THE CASE OF AN ORIGINAL DEPOSIT
Issued pursuant to Rule 7.1 of Budapest Treaty on the International Recognition of the
Deposit of Microorganisms for the Purposes of Patent Procedure

To: SEO, Jeong-min
NEOREGEN BOPTECH
89 Seoho-ro, Gwonseon-gu, Suwon city, Gyeonggi-do, Korea

I. IDENTIFICATION OF THE MICROORGANISM
Identification reference given by the
DEPOSITOR:
Lactobacillus plantarum L-14 | Accession number given by the
INTERNATIONAL DEPOSITARY
AUTHORITY:
KCTC 13497 BP
II. SCIENTIFIC DESCRIPTION AND/OR PROPOSED TAXONOMIC
DESIGNATION
The microorganism identified under I above was accompanied by:
( ) a scientific description
( ) a proposed taxonomic designation
(Mark with a cross where applicable)
III. RECEIPT AND ACCEPTANCE
This International Depositary Authority accepts the microorganism identified under I
above, which was received by March 15, 2018.
IV. RECEIPT OF REQUEST FOR CONVENRSION
The microorganism identified under I above was received by this International
Depositary Authority on ______________ (date of the original deposit) and a request to
convert the original deposit to a deposit under the Budapest Treaty was received by it on
(date of receipt of request for conversion).
V. INTERNATIONAL DEPOSITARY AUTHORITY
Name: Korean Collection for Type
Cultures (KCTC)

Address: Korea Research Institute of
Bioscience and Biotechnology (KRIBB)
Ipsin-gil, Jeongeup-shi, Jeolla-bukdo,
Korea (56212) | Signature(s) of person(s) having the power
to represent the International Depositary
Authority or of authorized official(s):
Director KIM, Cha-yeong

Date: March 15, 2018
Form BP/4 (KCTC Form 17)

## Claims

1. A pharmaceutical composition for preventing or treating a metabolic disease, comprising a *Lactobacillus plantarum*-derived polysaccharide or a *Lactobacillus plantarum* extract.

2. The pharmaceutical composition according to claim 1, wherein the *Lactobacillus plantarum* is at least one selected from the group consisting of *Lactobacillus plantarum* L-14 (accession number KCTC13497BP), *Lactobacillus plantarum* ATCC 10241, *Lactobacillus plantarum* NCDO704, and *Lactobacillus plantarum* NCDO1193.

3. The pharmaceutical composition according to claim 1, wherein the polysaccharide is at least one of an intracellular polysaccharide and an extracellular polysaccharide.

4. The pharmaceutical composition according to claim 1, wherein the polysaccharide is glucose.

5. The pharmaceutical composition according to claim 1, wherein the *Lactobacillus plantarum* extract is an ultrasonic crushed product of *Lactobacillus plantarum.*

6. The pharmaceutical composition according to claim 1, wherein the metabolic disease is at least one selected from the group consisting of insulin resistance, type 2 diabetes, hyperlipidemia, fatty liver, obesity and inflammation.

7. A food composition for preventing or improving a metabolic disease, comprising a *Lactobacillus plantarum-*derived polysaccharide or a *Lactobacillus plantarum* extract.

8. A method for preventing or treating a metabolic disease, comprising administering to an individual a *Lactobacillus plantarum-derived* polysaccharide or a *Lactobacillus plantarum* extract.
